Europäisches Patentamt

European Patent Office

Office européen des brevets

(11) Publication number : **0 501 779 A1**

## EUROPEAN PATENT APPLICATION

(21) Application number : 92301620.8

(22) Date of filing : 26.02.92

(51) Int. Cl.$^5$ : **C12P 21/08,** C12N 5/20,
C12N 15/06

(30) Priority : 01.03.91 US 663335

(43) Date of publication of application :
02.09.92 Bulletin 92/36

(84) Designated Contracting States :
AT BE CH DE DK ES FR GB GR IT LI NL PT SE

(71) Applicant : ELI LILLY AND COMPANY
307, East McCarty Street
Indianapolis Indiana 46285 (US)

(72) Inventor : Kramer, Ruth Maria
3740 Governos Road
Indianapolis, Indiana 46208 (US)
Inventor : Manetta, Joseph Vincent
8134 North Richardt Avenue
Indianapolis, Indiana 46256 (US)
Inventor : Sportsman, John Richard
4725 Clayburn Drive
Indianapolis, Indiana 46268 (US)

(74) Representative : Hudson, Christopher Mark et
al
Erl Wood Manor
Windlesham Surrey GU20 6PH (GB)

(54) Antibodies reactive with human cytosolic phospholipase A2.

(57) Novel monoclonal and polyclonal antibodies which selectively bind the approximately 100 Kda, human, cytosolic phospholipase $A_2$ enzyme are taught and claimed as part of the invention. The claimed antibodies are useful in a streamlined procedure for purifying $cPLA_2$. Another aspect of the invention is the use of the claimed antibodies as reagents for detecting the presence of $cPLA_2$ in biological fluids.

EP 0 501 779 A1

Jouve, 18, rue Saint-Denis, 75001 PARIS

The invention belongs to the general field of immunology and includes both monoclonal and polyclonal antibodies reactive with a human, 100 kDa cytosolic phospholipase $A_2$ ($cPLA_2$) enzyme. The invention also encompasses a method for purifying and identifying $cPLA_2$ using the claimed antibodies.

Human $cPLA_2$ and a method for purifying it is described in U.S. Patent Application Serial No. 07/573,513. Before the present invention, antibodies specific for $cPLA_2$ were unknown. Moreover, there was no facile method for purifying $cPLA_2$ to near homogeneity. Before this invention there also was no way to quickly and specifically identify the presence of $cPLA_2$ in complex biological mixtures. The present invention provides novel antibodies specific for $cPLA_2$, and in addition, the invention provides rapid methods for purifying and identifying $cPLA_2$.

Phospholipase $A_2$ ($PLA_2$) is the common name for phosphatide 2-acylhydrolase which catalyzes the hydrolysis of the sn-2-acyl ester bond of phosphoglycerides producing equimolar amounts of lysophospholipids and free fatty acids (Dennis, E. A., The Enzymes, Vol. 16, Academic Press, New York, (1983)). Phospholipase $A_2$ enzymes are found in all living species and form a diverse family of enzymes. Of those studied to date, the vast majority have a molecular weight of approximately 14 kDa, and their amino acid sequences show great homology.

The most abundant and commonly studied $PLA_2$ enzymes are the secreted forms. These enzymes are produced within the cell, packaged into secretory vesicles and, upon stimulation of the cells, released into the extracellular environment where they aid in the digestion of biological material. In contrast, $cPLA_2$ is found in vanishingly small amounts, remains within the cell and serves in an entirely different capacity than the secreted forms. Thorough investigation of intracellular $PLA_2$s has been hampered by the extremely low concentration of these enzymes in cells (Vadas and Pruzanski, Lab. Investigation, 55, 4:391 (1986)).

The ability to modulate receptor mediated $cPLA_2$ activity via specific inhibitors is a desirable goal and may lead to new therapies for the treatment of asthma, ischemia, arthritis, septic shock, and inflammatory diseases of the skin. The inactivation or specific inhibition of $cPLA_2$ activity associated with particular disease states will be of great use to the medical community. To accomplish this goal, $cPLA_2$ involved in the pathogenesis of certain diseases must be first identified and isolated. The specification describes how to prepare and isolate monoclonal and polyclonal antibodies to $cPLA_2$. For purposes of this application, the term antibody includes antibody fragments which retain their specific binding capacity. The specification will further show how to use antibodies to purify $cPLA_2$ from complex biological mixtures and how to identify the presence of the enzyme in various preparations.

The present invention encompasses antibodies reactive with $cPLA_2$ and a method of using such antibodies for isolating $cPLA_2$ comprising:

a) immobilizing said antibody onto a surface;

b) contacting said immobilized antibody with a mixture containing $cPLA_2$;

c) separating said immobilized antibody bound to $cPLA_2$ from said mixture;

d) recovering $cPLA_2$ by removing the antibody-bound $cPLA_2$ from said immobilized antibody.

The invention also includes a method of using such antibodies for identifying the presence of $cPLA_2$ comprising:

a) immobilizing said antibody onto an assay surface;

b) contacting said assay surface with a liquid suspected of containing $cPLA_2$ and washing said assay surface with a suitable solution;

c) contacting said assay surface with an anti-$cPLA_2$ antibody labeled with a reporting group and washing said assay surface with a suitable solution;

d) detecting the presence of said reporting group.

Figure 1 shows $cPLA_2$ reactivity of serum from an immunized mouse and a control mouse. The three titration curves clearly show that serum from the immunized mouse contains polyclonal antibodies reactive with $cPLA_2$ while the control serum does not.

Figure 2 shows the results of an immunoadsorption assay and clearly demonstrates that anti-$cPLA_2$ mabs and antiserum are able to remove $cPLA_2$ enzyme activity from a solution known to possess such activity. Column 1 is the negative control; column 2 is antiserum #132-T; column 3 is mab #132 PL12, and column 4 is mab #132 PC3-1.

Figure 3 shows the results of an immunoblot. Lane 1 is the negative control, and lane 2 is the positive control, antiserum #132-T. Lane 3 is mab #132 PL12 SC-D1, and lane 4 is mab #132 PC3-1 SC-E8.

The preparation of antibodies against proteins of interest is well known in the art. See Galfre and Milstein, Methods in Enzymology, Vol. 73, Academic Press, New York (1981); James W. Goding, Monoclonal Antibodies: Principles and Practice, Academic Press, Orlando, Florida (1986); Current Protocols in Molecular Biolopy, F. M. Ausubel, et al. ed., Wiley Interscience, New York, (1987).

To prepare antibodies reactive with a protein of interest, the protein must be first purified. Relatively crude antigenic preparations of the protein may be used for immunization purposes. However, highly purified protein is required to determine accurately if hybridomas are producing the sought after monoclonal antibodies (mabs) or to assay the antibody titers of immune serum. Thus, the following describes how to purify human $cPLA_2$ more than 10,000 fold to near homogeneity, how to prepare mabs and antiserum (polyclonal antibodies) against $cPLA_2$ and how to use the antibodies to purify human $cPLA_2$ in a single procedure.

Purification of $cPLA_2$:

A method for purifying $cPLA_2$ is described in U.S. Patent Application Serial No. 07/573,513 (European Patent Application No. 91307746.7). The following summarizes that method and briefly describes how $cPLA_2$ is isolated from the human leukemia monoblastoid cell line U937. the American Type Culture Collection (ATCC) and may be obtained under accession number CRL 1593. Once grown and harvested, U937 cells are lysed by nitrogen cavitation in the presence of protease inhibitors. A soluble fraction is prepared from the lysate by ultracentrifugation. The resulting solution of cytosolic proteins contains $cPLA_2$ and is subjected to a series of purification procedures.

The soluble fraction of the cell lysate is run through a series of column chromatography procedures. Anion exchange chromatography is followed by hydrophobic interaction, molecular sizing and finally another hydrophobic interaction technique where the conditions are such that the $cPLA_2$ binds the resin weakly. Each column is run individually, and the eluate is collected in fractions while monitoring for absorbance at 280 nm. The peak protein fractions are assayed for phospholipase activity, and those fractions with the desired activity are then run over the next column until a homogeneous solution of $cPLA_2$ is obtained.

Active $cPLA_2$ which has been purified to homogeneity may be stored at -20°C in a solution prepared by mixing one part of the final column buffer containing the $cPLA_2$, with two parts of 75% glycerol in water plus 2 mM 2-mercaptoethanol (2-ME). Under these conditions, the $cPLA_2$ retains enzymatic activity for a period of months.

Once the $cPLA_2$ has been isolated, antibodies specific for $cPLA_2$ may be raised by conventional methods that are well known in the art. Repeated injections into a host of choice over a period of weeks or months will elicit an immune response and result in significant anti-$cPLA_2$ serum titers. Preferred hosts are mammalian species and more highly preferred species are rabbits, goats, sheep and mice. Blood drawn from such immunized animals may be processed by established methods to obtain antiserum (polyclonal antibodies) reactive with $cPLA_2$. The antiserum may then be affinity purified by adsorption to $cPLA_2$ according to techniques known in the art. Affinity purified antiserum may be further purified by isolating the immunoglobulin fraction within the antiserum using procedures known in the art. The resulting material will be a heterogeneous population of immunoglobulins reactive with $cPLA_2$.

Anti-$cPLA_2$ antibodies may also be generated by preparing a semi-synthetic immunogen consisting of a synthetic peptide fragment of $cPLA_2$ conjugated to an immunogenic carrier. Numerous schemes and instruments useful for making peptide fragments are well know in the art. Many suitable immunogenic carriers such as bovine serum albumin or keyhole lympet hemocyanin are also well known in the art as are techniques for coupling the two proteins. Once the semi-synthetic immunogen has been constructed, the procedure for making antibodies specific for the $cPLA_2$ fragment is identical to those used for making antibodies reactive with natural $cPLA_2$.

Anti-$cPLA_2$ mabs are readily prepared using purified $cPLA_2$. Methods for producing mabs have been practiced for over 15 years and are well known to those of ordinary skill in the art. Repeated intraperitoneal or subcutaneous injections of $cPLA_2$ in adjuvant will elicit an immune response in most animals. Hyperimmunized B-lymphocytes are removed from the animal and fused with a suitable fusion partner cell line capable of being cultured indefinitely. Preferred animals whose B-lymphocytes may be hyperimmunized and used in the production of mabs are mammals. More preferred animals are rats and mice and most preferred is the balb/c mouse strain.

Numerous mammalian cell lines are suitable fusion partners for the production of hybridomas. Many such lines are commercially available from the ATCC and commercial suppliers. Preferred fusion partner cell lines are derived from mouse myelomas and the HL-1™ Friendly myeloma-653 cell line (Ventrex, Portland, ME) is most preferred.

Once fused, the resulting hybridomas are cultured in a selective growth medium for one to two weeks. Two well known selection systems are available for eliminating unfused myeloma cells, or fusions between myeloma cells, from the mixed hybridoma culture. The choice of selection system depends on the strain of mouse immunized and myeloma fusion partner used. The AAT selection system, described by Taggart and Samloff, Science 219, 1228 (1982), may be used; however, the HAT (hypoxanthine, aminopterin, thymidine) selection system, described by Littlefield, Science 145, 709 (1964), is preferred because of its compatibility with the preferred mouse strain and fusion partner mentioned above.

Spent growth medium is then screened for immunospecific mab secretion. Enzyme linked immunosorbant assay (ELISA) procedures are best suited for this purpose; though, radioimmune assays adapted for large volume screening are also acceptable. Multiple screens designed to consecutively pare down the considerable number of irrelevant or less desired cultures should be performed. Cultures that secrete mabs reactive with $cPLA_2$ should be screened for cross-reactivity with known $PLA_2$s. Mabs that preferentially bind to $cPLA_2$ are isotyped using commercially available assays. The preferred mabs are of the IgG class, and more highly preferred mabs are of the $IgG_1$ and $IgG_{2a}$ subisotypes.

Hybridoma cultures which secrete the sought-after mabs should be sub-cloned several times to establish monoclonality and stability. Well known methods for sub-cloning eukaryotic, non-adherent cell cultures include limiting dilution, soft agarose and fluorescence activated cell sorting techniques. After each subcloning, the resultant cultures must be re-assayed for antibody secretion and isotype to ensure that a stable antibody-secreting culture has been established.

The claimed anti-$cPLA_2$ antibodies are essential reagents for preparing an immunoaffinity surface necessary to practice the $cPLA_2$ isolation method of the invention. The immunoaffinity surface is formed by immobilizing anti-$cPLA_2$ antibodies to a substance so that at least some of the antibody binding site remains exposed and capable of binding its antigen. Substances ranging from porous polysaccharide based beads to plastic polymers to inorganic materials are substances to which the antibodies may be immobilized. Preferred substances are those which provide a maximal surface area to volume ratio and do not adversely affect the antibodies or $cPLA_2$. Polysaccharide matrices formed into various sized beads are more highly preferred because they are porous, provide high surface area to volume ratios, are easy to handle and are well known and understood in the biochemical purification art. See Affinity Chromatography Principles & Methods, Pharmacia Fine Chemicals, (1983).

A wide assortment of schemes for immobilizing antibodies has developed over the past few decades. Immobilization can be accomplished by covalently coupling the antibody directly to the desired substance or by bridging the antibody to the substance.

CNBr and carbodiimide coupling of antibodies to polysaccharide based beads such as Sepharose™ (Pharmacia, Pistcataway, NJ) are illustrative of direct coupling schemes that are consistent with the invention. Direct couplings generally do not orient the antibodies in any particular fashion; however, some types of direct couplings are able to reproducibly orient the antibody on the immobilizing substance.

Preferred coupling schemes orient the antibody such that its antigen binding regions remain exposed. One such scheme utilizes the natural carbohydrate found on the heavy chains of the antibody. By first oxidizing the carbohydrate moieties to the corresponding aldehydes then reacting the aldehyde with a primary amino group on the surface, it is possible to link the antibody in an advantageous orientation.

Many types of bridges are possible and include small organic linkers which covalently bind the antibody to the immobilizing substance. Such spacer arms are acceptable and preferably should not interact with proteins once the bridge has been formed.

Larger multivalent molecules bound to the immobilizing substance and which are capable of binding several antibodies describe another type of bridge. Specific immunoadsorbants bound to the immobilizing surface and which are capable of non-covalently binding the antibody represents yet another type of bridge. Protein-A is an example of a specific immunoadsorbant that is capable of orienting the antibody. By chemically bonding Protein-A to an immobilizing surface then allowing the antibodies to bind to it, the Fab antibody portions will be oriented away from the immunoaffinity surface resulting in a favorable configuration.

The above discussion is in no way meant to limit the scope of the invention. Numerous other well known schemes for linking antibodies to immobilizing substances are consistent with the invention.

Once the immunoaffinity surface has been prepared, the $cPLA_2$ containing liquid must be contacted with the surface. Batch methods are adequate, though column chromatography is preferable. Batch mode separation can be accomplished by filtering, centrifuging or decanting. When using column chromatography, a simple washing step serves to separate the mixture from the immobilized antibodies.

Removal of $cPLA_2$ from the immunoaffinity surface can be accomplished by subjecting the surface-bound $cPLA_2$ complex to a solution capable of disrupting the interactions between the antibody and $cPLA_2$. The solution preferably will not adversely affect the immunoaffinity surface or $cPLA_2$. More preferred solutions are buffered, isotonic, salt solutions at near neutral pH which contain millimolar concentrations of 2-ME. The most highly preferred solutions are those which do not adversely affect the enzymatic activity of $cPLA_2$.

It is well known that antibodies labeled with a reporting group can be used to identify the presence of antigens in a variety of milieus. Antibodies labeled with radioisotopes have been used for decades in radioimmune assays to identify, with great precision and sensitivity, the presence of antigens in a variety of biological fluids. More recently, enzyme labeled antibodies have been used as a substitute for radio-labeled antibodies in the popular ELISA.

Antibodies of the present invention can be bound to an immobilizing substance such as a polystyrene well or particle and used in immunoassays to determine whether $cPLA_2$ is present in a test sample. In this embodiment of the invention, a sample is contacted with the immunoaffinity surface and allowed to incubate. After a washing step, any $cPLA_2$ that has bound to the immunoaffinity surface is detected by contacting the surface with another antibody of the invention labeled with a reporting group.

In another mode of the assay embodiment, a test sample suspected of containing $cPLA_2$ is dried onto a surface, forming an immobilized test sample. A labeled antibody of the invention is then contacted with the immobilized test sample and allowed to incubate. If the sample contains $cPLA_2$, the labeled antibody will bind to the immobilized $cPLA_2$. This method can also be done using an unlabeled antibody of the invention followed by a labeled secondary antibody that binds to an antibody of the invention which has already bound to $cPLA_2$. After washing, the immobilized test sample is measured to detect the presence of any reporting groups.

Reporting groups are typically enzymes such as alkaline phosphatase, horseradish peroxidase or beta-D-galactosidase. Suitable substrates produce a color change when reacted with the enzyme. In so doing, measurements of the color intensity can be quantitated using a spectrophotometer. If the reporting group is a radioisotope, an appropriate gamma or beta ray detecting instrument can be used to quantitate the reporting group. The intensity of the reporting group directly correlates, with the amount of $cPLA_2$ in the test sample.

The following examples will help describe how the invention is practiced and will illustrate the characteristics of the claimed anti-$cPLA_2$ antibodies.

## EXAMPLE 1

### Culture of U937 Cells

The human leukemia monoblastoid cell line U937 was obtained from American Type Culture Collection (ATCC). This cell line is a permanent part of the collection and is available under accession number CRL 1593. U937 cells were maintained in culture in D-MEM/F-12 medium (Gibco Laboratories Life Technologies, Inc., Grand Island, NY) supplemented with 5% fetal bovine serum (HyClone Laboratories, Logan, Utah), 4 mM L-glutamine (Tanabe USA, San Diego, CA), 0.02% Pluronic F-68 (BASF Corporation, Parsippany, NJ), 1 mM $CaCl_2$, 50 ug/ml Tobramycin (Eli Lilly and Co., Indianapolis, IN), 20 mM Hepes (USB, Cleveland, OH), 2.2 g/l NaHCO3, 100 uM ethanolamine (Sigma Chemical, St. Louis, MO).

The ampule from ATCC containing 1 ml of cell suspension was removed from its dry ice container and placed into a 37°C water bath with vigorous agitation until the ice had melted. All operations from this point were carried out using sterile techniques. The ampule was opened, and the contents were removed and placed into a sterile 15 ml conical centrifuge tube. After adding 9 ml of medium (at 37°C), the tube was centrifuged at 1000 rpm for 10 minutes in a table top centrifuge (Damon/IEC Division, Needham Heights, MA). The medium was decanted and the cell pellet resuspended in 50 ml of medium (37°C) and transferred to a T-75 tissue culture flask (Corning Glass, Corning, NY) that was placed in a 37°C incubator equilibrated to 5% $CO_2$, 95% air.

When the cell count reached $1-1.5 \times 10^6$ cells/ml as determined by a Coulter Counter (Coulter Electronics, Hialeah, FL), the culture was transferred into 450 ml fresh growth medium (500 ml total) in a 500 ml sterile microcarrier spinner flask (Bellco Biotechnology, Vineland, NJ) and stirred at 50 rpm using a microcarrier magnetic stirrer (Bellco) in a 5% $CO_2$ incubator at 37°C. When cell density reached $1-1.5 \times 10^6$, the 500 ml cell suspension was placed into 5.5 liters of medium at 37°C. in a 6 liter microcarrier spinner (Bellco). The spinner flask was fitted with 0.2 micron vent filters (Gelman Sciences, Ann Arbor, MI) so that the headspace could be gassed continuously with 5% carbon dioxide, 25% oxygen, and 70% nitrogen. Stirring was at 50 rpm at 37°C. When cell density reached $1-1.5 \times 10^6$ cells/ml, the 5 liter cell suspension was placed into approximately 36 liters of medium at 37°C in a sterile 36 liter Biotech Overhead Drive System (Bellco). The culture was sparged with 70% nitrogen, 25% oxygen, and 5% carbon dioxide through a sterile vent filter. A Pasteur pipet was fitted through one of the side ports, and gas flow was set at 5 to 10 bubbles/second. The culture was stirred at 6-7 rpm and incubated at 37°C. Daily cell counts were performed and when cell density reached approximately $1 \times 10^6$ cells/ml, the culture was sparged at a very slow rate with 100% oxygen.

When the culture reached $2-3 \times 10^6$ cells/ml, the contents of the growth vessel were transferred to a sterile 50 liter carboy (Nalgene, Rochester, NY). The carboy was capped and stored at 4°C for approximately 48 hours to allow the cells to settle to the bottom of the container. All subsequent steps were performed at 4°C and did not require sterile procedures. Most of the medium was aspirated from the top of the carboy and discarded, leaving 5-6 liters of cell suspension. The carboy was swirled to resuspend the cells in the medium, and the cell suspension was poured into six, 1 liter centrifuge bottles (Nalgene). The bottles were centrifuged for 30 minutes at 2500 rpm in an IEC centrifuge (Damon/IEC). The supernatant was discarded, and the cells were washed in 2 liters of 140 mM NaCl, 5 mM KCl, 2 mM ethylenediamine tetraacetic acid (EDTA), 25 mM Tris/HCl at pH 7.4.

5

The washed cell pellets were combined and resuspended at $10^8$ cells/ml (approximately 10 mg/ml protein) in 150 mM NaCl, 2 mM EDTA, 50 mM Tris/HCl, 100 uM leupeptin at pH 8 yielding approximately 500 ml of cell suspension or 5 gm of cellular protein.

EXAMPLE 2

Purification of $cPLA_2$

After addition of phenylmethylsulphonyl fluoride (PMSF) and pepstatin A to 1 mM and 100 uM (from a combined stock solution in methanol of 200 mM and 20 mM, respectively), the cells were transferred to a precooled Parr cell disruption bomb (Parr Instr. Co., Moline, IL), mixed with a magnetic stirrer at 4°C for 20 minutes under 600 psi nitrogen and lysed by releasing the pressure. The cell lysate was centrifuged (150,000 x g, 60 min, 4°C) and the resulting supernatant collected. Prior to column chromatography this cell extract was filtered first through cheese cloth and then through a Millipak-40 filter (0.22 um, Millipore, Bedford, MA) using a variable speed peristaltic pump.

The cytosolic fraction (2-3 gm protein) was applied to a 3.2 x 14 cm Fast Flow Sepharose Q (Pharmacia) column that had been pre-equilibrated with 150 mM NaCl, 2 mM 2-ME, 1 mM ethylene glycol-bis(beta-aminoethyl ether) N,N,N',N'-tetraacetic acid (EGTA), 25 mM Tris/HCl, pH 8 at a flow rate of 6 ml/min. The column was washed with 500 ml of the same buffer, and elution proceeded by developing a 1500 ml linear salt gradient from 150 mM to 500 mM NaCl in 2 mM 2-ME, 1 mM EGTA, 25 mM Tris/HCl, pH 8 collecting fractions of 12 ml. The column eluate was monitored for absorbance at 280 nm (AUF 2.0) and 2 ul aliquots of each fraction were assayed for $PLA_2$ activity. The $PLA_2$ eluted with approximately 380 mM NaCl at fractions 47-56 (120 ml). These fractions were pooled and concentrated to 25 ml using an Amicon ultrafiltration stirred cell with a YM 30 membrane.

The concentrated peak fractions (150 mg of protein) from the anion exchange column were applied to a Phenyl-Superose HR 10/10 column (Pharmacia) that had been pre-equilibrated with 500 mM NaCl, 2 mM 2-ME, 1 mM EGTA, 25 mM Tris/HCl, pH 8 at a flow rate of 1 ml/min collecting 4 ml fractions. After washing the column with 120 ml of the same buffer, a 120 ml linear gradient was applied at a flow rate of 0.75 ml/min using one part of column buffer and one part of elution buffer (50% ethylene glycol, 2 mM 2-ME, 1 mM EGTA, 25 mM Tris/HCl, pM 8). The column eluate was monitored for absorbance at 280 nm and 2 ul aliquots of each fraction was assayed for $PLA_2$ activity. The $PLA_2$ eluted as a broad peak from 15-45% ethylene glycol in fractions 32-52 (40 ml). These fractions were pooled, concentrated to <1 ml using first an Amicon stirred cell with a YM 30 membrane and then Centricon-30 microconcentrators (Amicon), resuspended in 15 volumes of 30% ethylene glycol, 2 mM 2-ME, 150 mM NaCl, 1 mM EGTA, 25 mM Tris/HCl, pH 8 and re-concentrated to 2 ml.

The concentrated peak fractions from the hydrophobic interaction column were pooled (approximately 5 mg protein) and chromatographed on tandem Superose 12 columns (each 1.6 x 50 cm) pre-equilibrated with 30% ethylene glycol, 2 mM 2-ME, 150 mM NaCl, 1 mM EGTA, 25 mM Tris/HCl, pH 8 at a flow rate of 0.15 ml/min collecting 1.5 ml fractions. The column eluate was monitored for absorbance at 280 nm, and 2 ul aliquots of each fraction were assayed for $cPLA_2$ activity. The enzymatic activity eluted in fractions 38-47 (15 ml). These fractions were pooled and concentrated to <650 uls using 8 parallel Centricon-30 microconcentrators (Amicon). The concentrated samples (approximately 80 uls per concentrator) were each resuspended in 2.5 ml of 150 mM NaCl, 2 mM 2-ME, 1 m EGTA, 25 mM Tris/HCl, pH 8 (to give a final ethylene glycol concentration of <1 %) and reconcentrated to approximatelyl 80 uls, and then pooled resulting in a final volume of <650 uls.

The concentrated sample (approximately 1 mg protein) from the gel filtration column was diluted to 1 ml using 150 mM NaCl, 2 mM 2-ME, 1 mM EGTA, 25 mM Tris/HCl, pH 8. The sample was loaded onto a Phenyl-Superose HR 5/5 (Pharmacia) column that had been pre-equilibrated with the same buffer used for the dilution at a low rate of 0.25 ml/min, and 0.5 ml fractions were collected. After washing the column with 8 ml of the same buffer, a 10 ml linear gradient was applied at a flow rate of 0.25 ml/min using one part of column buffer and one part of elution buffer (50% ethylene glycol, 2 mM 2-ME, 1 mM EGTA, 25 mM Tris/HCl, pH 8). As discussed previously, elution is only necessary to recondition the column for subsequent use. The column eluate was monitored for absorbance at 280 nm, and a 2 ul aliquot of each fraction was assayed for $cPLA_2$ activity. The $cPLA_2$ activity appeared in fractions 7-20 (7 ml).

These fractions were pooled and concentrated to approximately 60 uls using first Centricon-30 microconcentrators (Amicon), then a Ultrafree-MC 10,000 NMWL filter unit (Millipore). The recovery of protein was approximately 100 ug.

## EXAMPLE 3

### cPLA$_2$, Enzymatic Activity Assay

The substrate, sonicated liposomes containing 1-palmitoyl-2-[14C]arachidonoyl-sn-glycero-3-phospho-choline ([14C]PC, 55 mCi/mmol from NEN Research Products) and sn-1,2-dioleoylglycerol (DG, Avanti Polar Lipids, Birmingham, AL) at a molar ratio of 2:1, was prepared as follows. [14C]PC (20 nmol, 1 x 10$^6$ dpm, 100 uCi/ml in toluene/ethanol) and DG (10 nmol, 100 ug/ml in chloroform) were dried under nitrogen. The lipids were dispersed in 1 ml of 150 mM NaCl, 50 mM Hepes, pH 7.5 (assay buffer) by sonication at 4°C with a Microson probe-sonicator (Heat-systems, Ultrasonics) for 4 x 15 second, with 45 second intervals. Bovine serum albumin (essentially fatty acid free, from a 100 mg/ml stock in water, Sigma) was added to a final concentration of 4 mg/ml. Samples to be assayed for cPLA$_2$ activity were incubated with 50 ul liposomes (0.5 nmol [14C]PC, 50,000 cpm containing 0.25 nmol of DG) in a total volume of 0.2 ml of assay buffer containing 1 mM CaCl$_2$ and 1 mM 2-ME. Incubations were carried out at 37°C for 15 minutes and terminated by adding 2 ml of Dole's reagent (2-propanol/heptane/0.5 M sulfuric acid, 40:10:1 containing 10 ug/ml of stearic acid). After mixing, 1.2 ml of heptane and 1 ml of water were added. The mixtures were briefly vortexed and the upper phase transferred to tubes containing 2 ml of heptane and 150 mg of Bio-Sil (Bio-Rad Laboratories) activated at 130°C before use. The tubes were thoroughly vortexed and centrifuged (1000 x g for 5 minutes). The supernatants were decanted into scintillation vials. After addition of 10 ml of a liquid scintillation cocktail (Ready Protein$^+$, Beckman) radioactivity was counted using a Packard liquid scintillation counter Model 1500. High radioactive counts correlate with enzymatic activity.

## EXAMPLE 4

### Preparation of Anti-cPLA$_2$ Monoclonal Antibodies

Cytosolic PLA$_2$ isolated according to the teachings of Examples 1 and 2 was used for all immunization and assay purposes. An 8 week old, balb/c female mouse was immunized intraperitoneally (i.p.) with 8 ug of cPLA$_2$ emulsified in Freund's complete adjuvant. The animal was given 3 i.p. boosts in Freund's incomplete adjuvant of 8 ug, 8 ug, and 4 ug at 3 week intervals. The final boost, 9 ug of cPLA$_2$ given i.p. without adjuvant, was performed 3 days prior to the fusion.

Three days following the final boost, the balb/c mouse was sacrificed and its spleen aseptically removed. The spleen was placed in a petri dish containing 5 ml of growth medium (GM); (10% fetal bovine serum (Hyclone, A-1111D), 45 % RPMI-1640 with HEPES (GIBCO, 380-2400AJ), 45% HL-1 (Ventrex, VBPG 001)). The splenocytes were teased from the organ using a blunt probe and then transferred to a 15 ml test tube with a total volume of 10 ml GM. The cells were allowed to stand at 22°C for 1 hour before transferring the suspended cells to another 15 ml test tube. A sample of the splenocytes was placed in 0.85% NH$_4$Cl and a cell count performed. The cells were then pelleted by centrifugation at approximately 200 x g for 5 min and then suspended in 5 ml of fresh GM.

HL-1™ Friendly myeloma-653 cells (Ventrex, VBPG 007 653) were used as the fusion partner for the cPLA$_2$ reactive splenocyte preparation. Prior to use, the cells were maintained in GM at a density to assure log phase growth. A cell count was performed and 19.7 X 10$^6$ cells were pelleted by centrifugation and resuspended in 5 ml fresh GM.

197 X 10$^6$ splenocytes in 5 ml of GM were combined with 19.7 X 10$^6$ myeloma cells and incubated 5 min at 37°C. The cells were then pelleted by centrifugation and all liquid removed. The cells were then loosened by gentle tapping. 1.5 ml of fusing media (43% PEG 4000 (ATCC) in RPMI-1640 with HEPES) was added at 37°C over a 1 min interval and then stirred for 1 min. RPMI-1640 with HEPES warmed to 37°C was added at a rate of approximately 1 ml/min for 2 min and then 8 ml/min for 1 min with constant agitation. The cells were then pelleted by centrifugation. The liquid was aspirated, and the pellet was loosened by gently tapping the tube.

The cells from the fusion were suspended in 200 ml of 10% hybridoma enhancing supplement (Sigma, H 8142), 20% fetal bovine serum (Hyclone), 35% RPMI-1640 w/ HEPES (GIBCO), 35% HL-1 (Ventrex), 1X HAT (Sigma; 5 mM hypoxanthine, 0.02 mM aminopterin, 0.8 mM thymidine). The cells were plated into 96 well cluster dishes at 200 uls per well and placed in a humidified 37°C incubator with a 5 % CO$_2$ atmosphere.

## EXAMPLE 5

CPLA$_2$ ELISA: Selection of Anti-cPLA$_2$ Secreting Hybridomas

After macroscopic colonies became visible (7-10 days), 50 uls of spent tissue culture medium were removed aseptically from each culture and assayed for cPLA$_2$ reactivity using an ELISA method. Assay plates were prepared by coating polystyrene microtiter plate wells with 100 uls of a 500 ng/ml solution of cPLA$_2$ in TBS (20 mM Tris pH 7.4, 0.15 M NaCl) plus 50 ug/ml BSA (Sigma) and incubating overnight at 4°C. The plates were then washed once with wash buffer (WB), 0.05% Tween 20 (Sigma) in TBS. The plates were then blocked with a 0.5% milk solution in TBS. 50 uls of spent medium from each growing culture was added to an assay well and incubated at 22°C for 1 hour. The plates were then washed 4X with WB. 50 uls of peroxidase labeled goat anti-mouse IgG, gamma chain specific (Sigma, A-3673) at a 1:500 dilution in TBS plus 1 mg/ml BSA, was added to each well and incubated 1 hour at 22°C. The plates were then washed 4X with WB. 50 uls of OPD substrate (Sigma, P-6912) in pH 5 citrate buffer with 0.012% hydrogen peroxide was added to develop the assay. After 20 min, the reaction was stopped with 50 uls of 1 N HCl, and the absorbance at 490 nm was determined using a 96 well plate reader. From the 960 wells seeded after fusion, spent growth medium from two culture wells generated absorbance readings at least 0.2 over background and were considered positive in the initial screen for cPLA$_2$ reactivity. These two parental hybridoma cultures and their corresponding antibodies were designated #132 PL12 and #132 PC3-1.

## EXAMPLE 6

Preparation of anti-cPLA2 Antiserum

An 8 week old, balb/c female mouse was given an i.p. injection of 8 ug of cPLA$_2$, as described in Examples 1 and 2, emulsified in Freund's complete adjuvant. The animal was given 3 i.p. boosts in Freund's incomplete adjuvant of 8 ug, 8 ug, and 4 ug at 3 week intervals. The last i.p. injection of 9 ug was given without adjuvant. Several bleeds were taken and later assayed for cPLA$_2$ reactivity. Bleed #132-1 was taken 4 weeks after the initial immunization, and bleed #132-T was taken 12 weeks after the initial immunization.

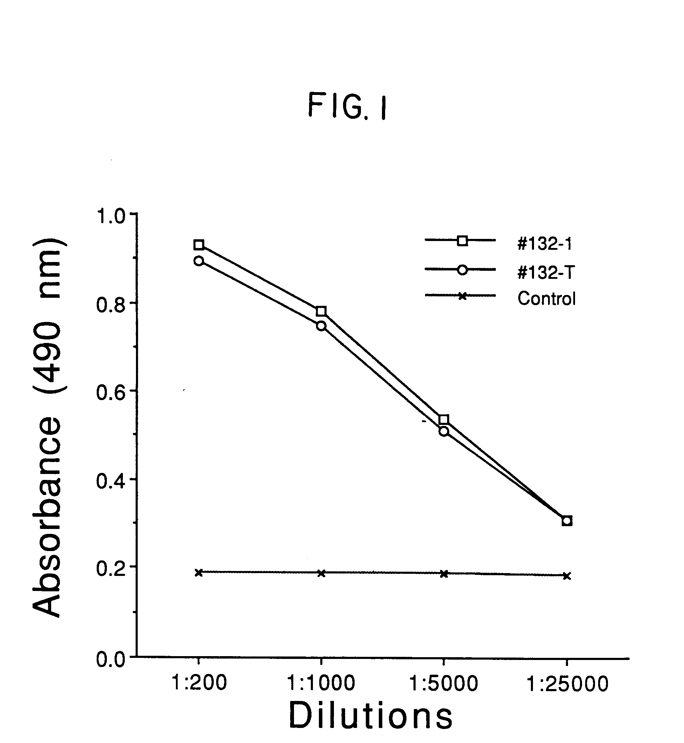

Serum was prepared from the bleeds and titrated against cPLA$_2$ in an ELISA. The ELISA was performed in accordance with Example 5, and the results are summarized in Figure 1. The graph in Figure 1 compares cPLA$_2$ reactivity of serum from the immunized mouse and a control mouse. The three titration curves clearly show that serum from the immunized mouse contains polyclonal antibodies reactive with cPLA$_2$ while the control serum does not.

## EXAMPLE 7

Immunoadsorption of cPLA$_2$ Enzymatic Activity

Spent medium from hybridomas #132 PL12, #132 PC3-1 and antiserum #132-T were assayed for the ability to deplete cPLA$_2$ activity from a known cPLA$_2$ solution. Spent medium from a hybridoma secreting an irrelevant antibody was used as the negative control. 100 uls of Fluorotec anti-mouse IgG beads (Baxter-Pandex, Mundelein, IL) were used to immobilize the antibody from 400 uls of spent hybridoma culture medium or serum. The mixture was incubated at 22°C overnight with gentle rocking. The beads were then pelleted by centrifugation and the liquid discarded. The beads were then washed with 1 ml of TBS with 1 mg/ml BSA to remove any unbound antibody. Then 100 uls of a 1.25 ug/ml (in 1 mg/ml BSA) cPLA$_2$ solution were added to the pellets and allowed to incubate for 4 hours at 22°C. The beads were then pelleted and the liquid was assayed for cPLA$_2$ activity, according to Example 3. The beads were further washed to remove any unbound cPLA$_2$ and then assayed for cPLA$_2$ activity.

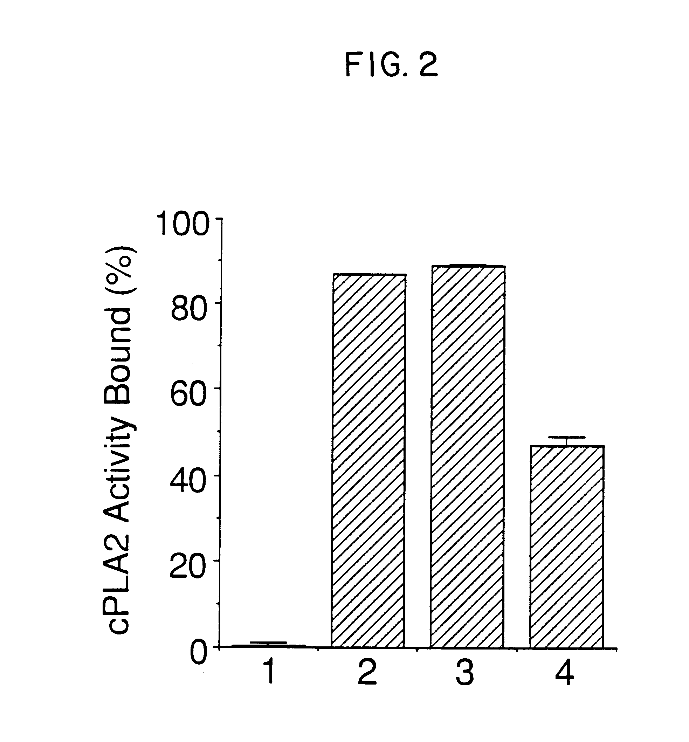

Figure 2 shows the results of the assay and clearly demonstrates that both mabs and the antiserum are able to remove cPLA$_2$ enzyme activity from the known enzymatic solution. Column 1 is the negative control; column 2 is antiserum #132-T; column 3 is mab #132 PL12, and column 4 is mab #132 PC3-1. The reader should note that mab #132 PC3-1 shows a diminished capacity to reduce enzyme activity suggesting that it binds an epitope on the cPLA$_2$ molecule that is not readily accessible in the native cPLA$_2$ conformation.

Thus, both mabs and the antiserum were confirmed to be cPLA$_2$ reactive by their ability to remove cPLA$_2$ enzyme activity from the known solution. The two hybridoma cultures were then subcloned by limiting dilution, reassayed for cPLA$_2$ reactivity and sub-isotyped using a commercially available kit. The resulting monoclonal antibody secreting hybridomas were designated #132 PL12 SC-D1 and #132 PC3-1 SC-E8. Each culture secreted a mab reactive with cPLA$_2$. Mab #132 PL12 SC-D1 was found to be in the IgG$_1$ sub-class, and mab #132 PC3-1 SC-E8 was found to be in the IgG$_{2a}$ sub-class.

## EXAMPLE 8

### Crossreactivity Analysis

An ELISA was performed according to the method described in Example 5 to determine if two protein-A purified mabs and an antiserum of the invention were crossreactive with two well-known, low molecular weight, secreted forms of PLA$_2$. Three types of assay wells, each containing a different type of PLA$_2$, were prepared. The three types of PLA$_2$ used as targets in the ELISA were cPLA$_2$ as described in Examples 1 and 2, secretory synovial PLA$_2$ isolated from rheumatoid arthritic synovial fluid (sPLA$_2$; Kramer et al., J. Biolopical Chem. 264, 5768 (1989)) and PLA$_2$ isolated from cobra (Naja naja) venom (Sigma).

The antibodies tested were mabs #132 PL12 SC-D1 and #132 PC3-1 SC-E8, described in Examples 6 and 7, and antiserum #132-1, described in Example 6. Control antibodies used in the experiment were protein-A purified mabs, #129 C2 and #129 D3, and antiserum, #128-T, which were raised against sPLA$_2$. Each sample was tested at a maximal binding concentration that was previously determined by titration.

The ELISA was performed in duplicate, the results of which are summarized in Table 1. The listed values are averages of the absorbance readings obtained for each assay well.

## Table  1

| Sample  # | Target | | |
|---|---|---|---|
| | cPLA2 | sPLA2 | Naja |
| 132-1  (1:2000  dilution) | 0.791 | 0.054 | 0.053 |
| 132 PL12 SC-D1  (8 ug/ml) | 0.579 | 0.010 | 0.008 |
| 132 PC3-1 SC-E8  (2.6 ug/ml) | 0.975 | 0.017 | 0.013 |
| 128-T  (1:2000 dilution) | 0.021 | 0.638 | 0.014 |
| 129 C2  (21 ug/ml | 0.025 | 0.336 | 0.017 |
| 129 D3 (3 ug/ml) | 0.022 | 0.564 | 0.017 |

Table 1 demonstrates that the antibodies of the invention, both monoclonal and polyclonal, specifically react with cPLA$_2$ and do not crossreact with two low molecular weight forms of PLA$_2$.

## EXAMPLE 9

### Immunoaffinity Chromatoghraphy

ImmunoPure$^R$ Ag/Ab Immobilization Kit #3 was purchased from Pierce (P.O. Box 117, Rockford, IL 61105). The kit contained all the reagents for producing the antibody affinity column except for the anti-cPLA$_2$ monoclonal antibody. The carbohydrate moieties on 0.8 mgs of protein-A purified antibody #132 PL12 SC-D1 were oxidized using sodium meta-periodate. Oxidized antibody was then reacted with the agarose beads provided in the kit. An 80% coupling affinity was measured by absorbance at 280 nm (final OD divided by starting OD X 100). Approximately half of the immunoaffinity beads were used to pour a 1 ml column that was equilibrated with approximately 10 mls of buffer A (1 mg/ml bovine serum albumin (BSA), 0.04 M Tris pH 7.4, 0.15 M NaCl, 2 mM 2-ME).

Approximately 6 mls of U937 high-speed supernatant (as described in Example 2A) was applied to the above-described immunoaffinity column at 0.05 ml/min at room temperature (4°C is preferred). Thereafter, the column was washed with approximately 4 mls of buffer A. The bound cPLA$_2$ was eluted using a 100 minute linear gradient (0.2 ml/min) beginning with 100% buffer A and ending with 100% buffer B (1 mg/ml BSA, 0.04 M Tris pH 7.4, 0.15 M NaCl, 2 mM 2-ME, 2.0 M MgCl$_2$). Approximately 2 ml fractions were collected and pooled into five groups and assayed for enzymatic activity according to Example 3.

Because cPLA$_2$ is such a rare enzyme, only minute amounts were recovered during immunoaffinity purification. To detect enzymatic activity in the eluate, it was necessary to include BSA as a carrier protein in the elution buffer. Those skilled in the art will recognize that adding an extraneous carrier protein to the freshly purified cPLA$_2$ is undesirable when larger amounts of the enzyme are purified.

Table 2 lists the results of the immunoaffinity purification. The final results are shown in the last column and are expressed as a percentage of the enzymatic activity in the prepurified sample. Over 80% of the total cPLA$_2$ activity found in the starting sample bound to the column and was recovered as active enzyme.

## Table 2

| Fraction # | Volume (ml) | Counts per Sample Size | Total Counts | Percent of Original |
|---|---|---|---|---|
| Load | 6.0 | 3575/1 ul | 21.5E6 | 100.0 |
| Flow Through | 12.5 | 2303/20 ul | 1.44E6 | 6.7 |
| 1 - 3 | 6.0 | 2184/20 ul | 0.65E6 | 3.0 |
| 4 - 6 | 6.0 | 1536/2 ul | 4.61E6 | 21.4 |
| 7 - 9 | 6.0 | 3918/2 ul | 11.75E6 | 54.7 |
| 10 - 12 | 6.0 | 2027/20 ul | 0.61E6 | 2.8 |

EXAMPLE 10

Western Blot Analysis:

Approximately 80 ngs of cPLA$_2$, purified as described in Example 2, was loaded onto each of 4 lanes of an 8-16% SDS gel. The gel was run at 25 mA until the dye reached the bottom of the gel. The protein in the gel was transferred to nitrocellulose (Novex, P.O.Box 1158, Encintas, CA 92024) using a Sartoblot (Sartorius Filters, Inc., 30940 San Clemente St., Hayward, CA 94544) apparatus and the procedure supplied with the instrument. The transfer conditions were 30 V constant for 1.5 hours. The development of the blot used rinse buffer (0.01M Tris pH 7.5, 0.001M EDTA, 0.15M NaCl, 0.1% Triton X-100) as its central buffering system.

The protein-containing nitrocellulose was cut into 4 strips corresponding to the SDS gel lanes. Each strip was incubated separately at 4°C overnight in 5 mls of a primary antibody solution. The primary antibody solutions were spent growth medium from mabs #132 PC3-1 SC-E8 and #132 PL-12 SC-D1. Antiserum 132-T, described in Example 6, at a 1:500 dilution in 0.3% BSA-rinse buffer was used as a positive control. and 0.3% BSA-rinse buffer alone served as the negative control. Each strip was then washed 4 times for 10 minutes per wash in rinse buffer. The strips were then incubated at room temperature for 4 hours in approximately 30 mls of a 1:250 dilution of I$^{125}$-labeled sheep anti-mouse Ig (Amersham Corp., Arlington Heights, IL). Finally the strips were washed 4 times as before and placed side by side in their pre-cut state under a piece of X-ray film in an autorad cassette. The film was exposed to the strips at -70°C for 16 hours after which the film was removed and developed.

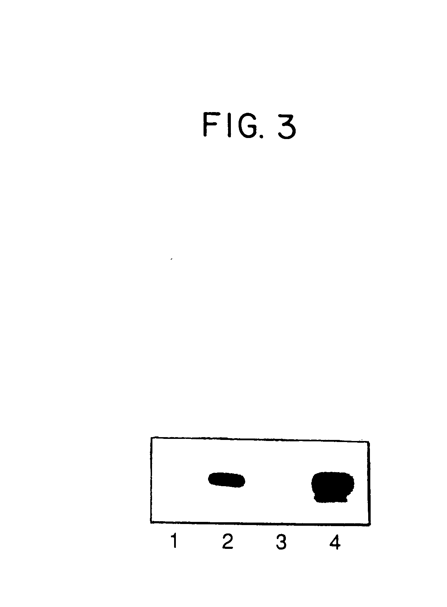

Figure 3 shows the results of the immunoblot. Lane 1 is the negative control, and lane 2 is the positive control, antiserum #132-T. Lane 3 is mab #132 PL12 SC-D1, and lane 4 is mab #132 PC3-1 SC-E8. The reader should note that lane 3 does not show a band which implies that mab #132 PL12 SC-D1 is not able to bind cPLA$_2$ in its denatured state.

When comparing figures 2 and 3, it is evident that mab #132 PL12 SC-D1 is best able to bind cPLA$_2$ in its native conformation indicating that this mab is preferable to use when purifying cPLA$_2$ in its active, native state. Conversely, mab #132 PC3-1 SC-E8 is best able to bind cPLA$_2$ in a denatured state.

Hybridoma #132 PL12 SC-D1 (Depositor Reference Id. Z48-5MG-63) and hybridoma #132 PC3-1-SC-E8 (Depositor Reference Id. Z48-5MG-70) were deposited with the ATCC under the terms of the Budapest Treaty under accession numbers HB 10563 and HB 10564 respectively.

## Claims

1. An antibody that specifically binds to human cytosolic phospholipase $A_2$ ($cPLA_2$).

2. The antibody of Claim 1 which is a monoclonal antibody.

3. The antibody of Claim 1 which is a polyclonal antibody.

4. Hybridoma #132 PL12 SC-D1 which is on deposit with the ATCC under the accession number HB10563.

5. The monoclonal antibody secreted by the hybridoma of Claim 4.

6. Hybridoma #132 PC3-1 SC-E8 which is on deposit with the ATCC under the accession number HB10564.

7. The monoclonal antibody secreted by the hybridoma of Claim 6.

8. An antibody as claimed in any one of Claims 1-3, 5 and 7 for use as an agent for purifying or identifying $cPLA_2$.

9. A process for preparing a $cPLA_2$-binding monoclonal antibody as claimed in any one of Claims 1-3, 5 and 7, comprising immunizing an animal B-cell with $cPLA_2$, fusing the immunized B-cell with a suitable myeloma cell, culturing the fused cell and collecting the monoclonal antibody. immunizing an animal with $cPLA_2$.

10. A process for preparing a $cPLA_2$-binding polyclonal antibody as claimed in any one of Claims 1 and 3, comprising immunizing an animal with $cPLA_2$.

## Claims for the followings Contracting State:ES

1. A process for preparing a polyclonal antibody that specifically binds to human cytosolic phospholipase $A_2$ ($cPLA_2$) comprising.immunizing an animal with $cPLA_2$.

2. A process for preparing a monoclonal antibody that specifically binds to human cytosolic phospholipase $A_2$($cPLA_2$) comprising.immunizing an animal B-cell with $cPLA_2$, fusing the immunized B-cell with a suitable myeloma cell, culturing the fused cell and collecting the monoclonal antibody.

3. A process according to Claim 2 for preparing the monoclonal antibody secreted by hybridoma #132 PL12 SC-D1 which is on deposit with the ATCC under the accession number HB10563.

4. A process according to Claim 2 for preparing the monoclonal antibody secreted by hybridoma #132 PC3-SC-E8 which is on deposit with the ATCC under the accession number HB10564.

## Claims for the following Contracting State: GR

1. A process for preparing a polyclonal antibody that specifically binds to human cytosolic phospholipase $A_2$ ($cPLA_2$) comprising.immunizing an animal with $cPLA_2$.

2. A process for preparing a monoclonal antibody that specifically binds to human cytosolic phospholipase $A_2$ ($cPLA_2$) comprising.immunizing an animal B-cell with $cPLA_2$, fusing the immunized B-cell with a suitable myeloma cell, culturing the fused cell and collecting the monoclonal antibody.

3. A process according to Claim 2 for preparing the monoclonal antibody secreted by hybridoma #132 PL12 SC-D1 which is on deposit with the ATCC under the accession number HB10563.

4. A process according to Claim 2 for preparing the monoclonal antibody secreted by hybridoma #132 PC3-SC-E8 which is on deposit with the ATCC under the accession number HB10564.

5. Hybridoma #132 PL12 SC-D1 which is on deposit with the ATCC under the accession number HB10563.

6. The monoclonal antibody secreted by the hybridoma of Claim 4.

# FIG. I

FIG. 2

# FIG. 3

1    2    3    4

**European Patent Office**

# EUROPEAN SEARCH REPORT

Application Number

EP 92 30 1620

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | JOURNAL OF BIOCHEMISTRY vol. 108, no. 6, December 1990, TOKYO, JAPAN pages 903 - 906; D. KIM ET AL.: 'Detection and subcellular localization of rabbit platelet phospholipase A2 which preferentially hydrolyzes an arachidonoyl residue.' * the whole document * --- | 1-10 | C12P21/08 C12N5/20 C12N15/06 |
| A | EUROPEAN JOURNAL OF BIOCHEMISTRY vol. 164, no. 1, 1987, BERLIN, GERMANY pages 129 - 135; J. DE JONG ET AL.: 'Monoclonal antibodies against an intracellular phospholipase A2 from rat liver and their cross-reactivity with other phospholipases A2.' * the whole document * --- | 1-10 | |
| X,P | THE JOURNAL OF BIOLOGICAL CHEMISTRY vol. 266, no. 8, 15 March 1991, BALTIMORE, USA pages 5268 - 5272; R. KRAMER ET AL.: 'The Ca2+-sensitive cytosolic phospholipase A2 is a 100-kDa protein in human monoblast U937 cells.' * the whole document * --- | 1-10 | TECHNICAL FIELDS SEARCHED (Int. Cl.5) C07K C12P C12N |
| X,P | THE JOURNAL OF BIOLOGICAL CHEMISTRY vol. 266, no. 17, 15 June 1991, BALTIMORE, USA pages 11366 - 11371; C. LESLIE: 'Kinetic properties of a high molecular mass arachidonoyl-hydrolyzing phospholipase A2 that exhibits lysophospholipase activity.' * the whole document * ----- | 1-10 | |

The present search report has been drawn up for all claims

| Place of search | Date of completion of the search | Examiner |
|---|---|---|
| THE HAGUE | 22 MAY 1992 | NOOIJ F.J.M. |

EPO FORM 1503 03.82 (P0401)